# EUROPEAN PATENT SPECIFICATION

(11) **EP 4 590 186 B1**
(45) Date of publication and mention of the grant of the patent: **24.06.2026**
(21) Application number: 23777384.1
(22) Date of filing: 22.09.2023
(51) Int. Cl.: A61B 5/024, A61B 5/1455, A61B 5/00

(54) **HEAD-MOUNTED MONITORING SYSTEM**
KOPFMONTIERTES ÜBERWACHUNGSSYSTEM
SYSTÈME DE SURVEILLANCE MONTÉ SUR LA TÊTE

(30) Priority: 23.09.2022 EP 22197550
(43) Date of publication of application: 30.07.2025
(73) Proprietor: Nederlandse Organisatie voor toegepast- natuurwetenschappelijk onderzoek TNO, 2595 DA 's-Gravenhage (NL)
(72) Inventor: AMELINK, Arjen, 2595 DA 's-Gravenhage (NL); ODERWALD, Michiel Peter, 2595 DA 's-Gravenhage (NL); GROEN, Eric Lysander, 2595 DA 's-Gravenhage (NL); BUSSINK, Peter Gerhardus Wilhelmus, 2595 DA 's-Gravenhage (NL)
(74) Representative: V.O.
(86) International application number: PCT/NL2023/050494
(87) International publication number: WO 2024/063651

(56) References cited:
- WO-A2-2006/086010
- WO-A2-2014/155288
- US-A1- 2016 270 656
- US-H- H1 039

## Description

### TECHNICAL FIELD AND BACKGROUND

The present disclosure relates to head-mounted monitoring systems, e.g. integrated as part of a head-mounted display system, and methods for monitoring a physiological parameter of a user, e.g. while operating a vehicle.

When operating a vehicle, it is important that the user stays alert and vigilant. For example, a pilot may suffer from (temporary) impairment while operating an airplane, especially in military aviation. Unmonitored or uncontrolled impairment of the pilot's physiological conditions may have disastrous consequences. For example, a pilot may suffer from an unexpected loss of consciousness due to hypoxia and/or a drop in blood pressure, e.g. induced by G-forces experienced during flight. Although the pilot's physiology may be measured during high-G training in a centrifuge and during hypoxia training in a hypobaric chamber, typical biosensors used for this purpose may be cumbersome or unsuitable to be continuously worn during flight. For example, near infrared spectroscopy (NIRS) sensors attached to the skin may hinder the pilot, and their signals may be polluted depending on underlying tissue.

As background, WO2014155288A2 describes a method and apparatus for head worn display with multiple exit pupils; USH1039H describes intrusion-free physiological condition monitoring; US2016270656A1 describes methods and systems for diagnosing and treating health ailments; WO2006086010A2 describes methods and devices for countering gravity induced loss of consciousness and pulse oximeter probes.

There is yet a need to more conveniently and accurately monitor, e.g. continuously measure, physiological parameters of users such as pilots and other drivers while operating an airplane or other vehicle.

### SUMMARY

One aspect the present disclosure provides a head-mounted monitoring (HMM) system. The system comprises a head-mount configured to attach the HMM system to a user's head, a light source configured to project source light onto a user's retina when the HMM system is attached to the user's head by the head-mount, and a sensor device configured to measure reflected light reflected from the user's retina. A computing device is configured to continuously monitor, a physiological parameter of the user
based on the measured light. Advantageously, the head-mount may allow the user to continuously wear the system while performing other activities such as operating a vehicle or other interaction with the environment. Accordingly, the user may freely move their head while the HMM system continuously monitors the user's (physiological) condition.

Another or further aspect of the present disclosure provides a head-mounted display (HMD). The HMD system may comprise the HMM system in addition to a display generator configured to generate a visible image representing informational content. and a display projection system configured to project the visible image onto the user's retina for conveying the informational content to the user. Advantageously, the sensor device of the HMM system can be configured to measure light reflected from the user's retina using at least part of the display projection system.

The inventors have recognized that, especially in military aviation, it is customary that the pilot receives visual information via a head-mounted display, also known as HMD. The HMD typically comprises a (semi) transparent display or reflecting window capable of presenting the information without requiring the user to look away from their usual viewpoints. For example, the HMD may be worn, e.g. integrated in front of a helmet (also known as a helmet-mounted-display); or part of a separate (see-through) display panel, e.g. mounted in front of, or projected onto, a front view window.

Furthermore, the inventors have recognized that by integrating a system for monitoring the user's physiological conditions in the existing HMD system, e.g. as part of a helmet, it is not necessary for the user to connect any separate biosensors. In a synergetic approach, the inventors have integrated part of the HMD's projection system with a monitoring system capable of measuring light that is reflected off the user's retina. A sensor device configured to measure light reflected back from the user's retina may use at least part of the same projection system that projects the HMD image into the user's eye. Advantageously, since the HMD system may already be used to continuously project light onto the retina, the inventors have recognized that the reverse optical path may be similarly continuously available for physiological readout by techniques such as retinal oxygenation measurement. For example, the reflected light may include visible light, e.g. originating from the HMD image and/or (more preferably) invisible light, e.g. infrared light which may originate from the display generator, or a dedicated infrared light source.

Without being bound by theory, an amount or (relative) intensity of the reflected light (preferably including scattered light), e.g. as function of wavelength and/or time, may depend on a (variable) absorption of the light in the retinal tissue, e.g. blood vessels. Advantageously, the retina may contain physiological information that is very similar to the cerebral physiology. In particular, the absorption and/or reflection of the light may depend on one or more physiological conditions of the user such as oxygen saturation in the blood, blood pressure, heart rate, et cetera. By correlating the measurement of reflected light with respective conditions or reference measurements, a physiological parameter may be determined, e.g. indicating a measure of one or more of the physiological conditions. For example, the physiological parameter may be provided as feedback to the user and/or used to control other systems such as flight control.

### BRIEF DESCRIPTION OF DRAWINGS

These and other features, aspects, and advantages of the apparatus, systems and methods of the present disclosure will become better understood from the following description, appended claims, and accompanying drawing wherein:
FIG 1 illustrates a head-mounted monitoring system;
FIG 2 illustrates a head-mounted display system with monitoring functionality;
FIGs 3A and 3B illustrate filtering of background light;
FIGs 4A-4C illustrate integrated devices for generation and measuring of infrared and/or visible light;
FIG 5A illustrates absorption spectra which may help to determine oxygenation of blood;
FIG 5B illustrates a measurement of reflected light as function of time, and at different wavelengths;
FIG 6A illustrates a ratio of light intensity measured at different wavelengths;
FIG 6B illustrates a correlation between the ratio of light intensity and oxygenation level measured by an independent sensor.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

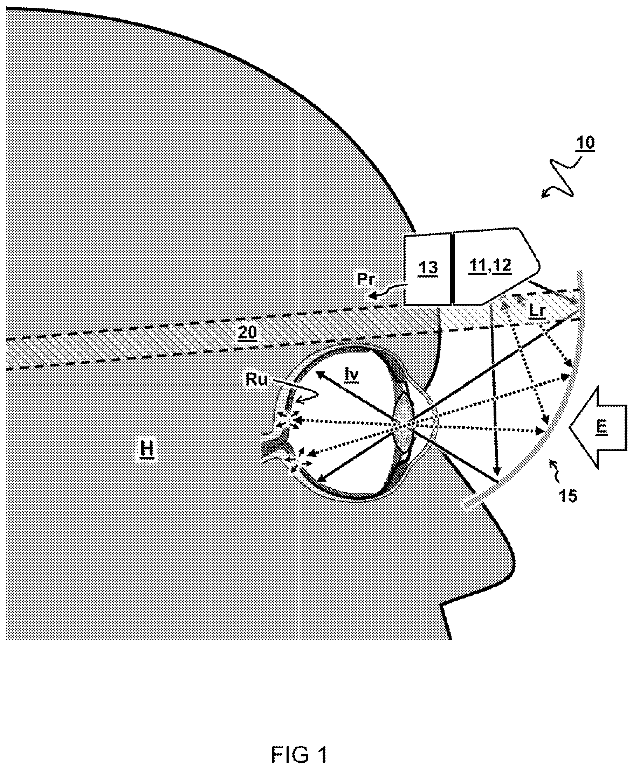

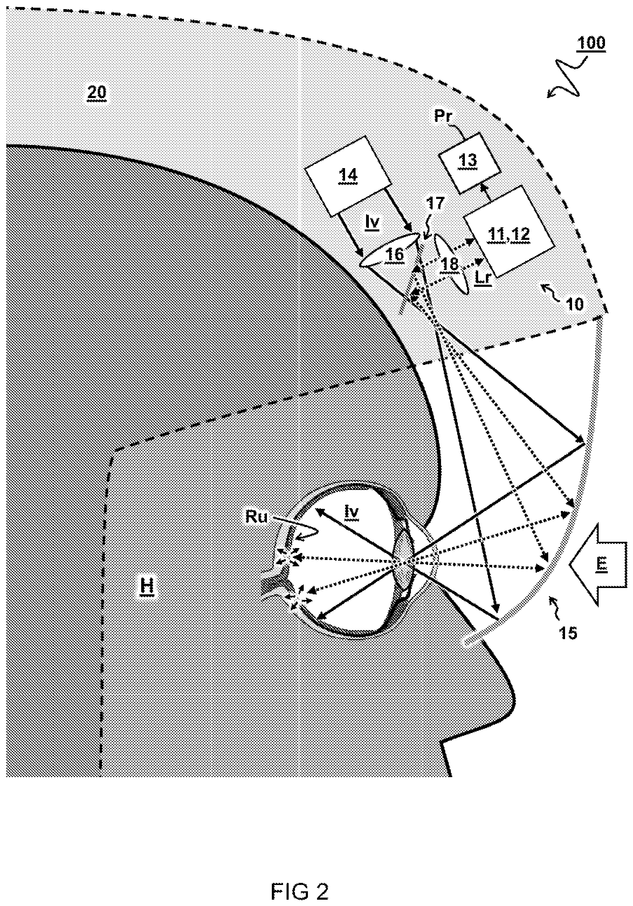

FIG 1 illustrates a head-mounted monitoring (HMM) system 10. FIG 2 illustrates a head-mounted display (HMD) system 100 which may incorporate the HMM system 10.

In some embodiments, e.g. as shown in FIGs 1 and 2, the HMM system 10 and/or HMD system 100 comprises a head-mount 20 configured to attach, e.g. secure, the system to a user's head "H". In other words, the head-mount 20 is configured to allow a user to wear the system on their head. For example, this allows the user to move their head, while the system follows their head movement. Preferably, the head-mount 20 is configured to, in use, carry at least part of the HMM or HMD system on the user's head, in particular with part of the wearable system arranged in front of the user's eye(s).

In one embodiment, e.g. as shown in FIG 1, the head-mount 20 comprises a set of ear hooks and/or nose support so that the system can be worn like spectacles or glasses (or monocle). In another or further embodiment, e.g. as shown in FIG 2, the head-mount 20 comprises a helmet which can be worn on the user's head. While FIG 1 illustrates a spectacle type head mount to carry an HMM system, and FIG 2 illustrates a helmet type head mount to carry a HMD system, these types of head mount can also be reversed. Also other or further head-mounting means and/or means for securing the respective system to the user's head can be envisaged such as one or more (head) straps, (head) clamps, a head band, a (soft) hat, et cetera.

In some embodiments, the system comprises or couples to a light source 11 configured to project source light Ls onto a user's retina "Ru", at least when the system is attached to the user's head "H" by the head-mount 20. For example, the light source 11 is integrated with and/or carried via the head-mount 20, or otherwise coupled to the head-mount. In one embodiment, e.g. as shown in FIG 1, the light source 11 is integrated with the spectacles. In another or further embodiment, e.g. as shown in FIG 2, the light source 11 is integrated in the helmet. Preferably, the light source 11 comprises an infrared light source. Alternatively, or additionally visible source light can be used. In another or further embodiment (not shown), the head-mount 20 is provided with a light guide configured to guide the source light Ls from a separate light source 11 to the user's retina "Ru". Although the signal strength may be relatively low and unpredictable, in principle, it can also be envisaged to omit the light source 11 and rely on environmental lighting alone; or in case of the HMD device, rely on lighting provided by the display generator 14.

In some embodiments, the system comprises a sensor device 12 configured to measure reflected light "Lr" reflected from the user's retina "Ru". Preferably, the sensor device 12 is integrated with and/or carried via the head-mount 20, or otherwise coupled to the head-mount. In one embodiment, e.g. as shown in FIG 1, the sensor device 12 is integrated with the spectacles. In another or further embodiment, e.g. as shown in FIG 2, the sensor device 12 is integrated in the helmet. In another or further embodiment (not shown), the head-mount 20 is provided with a light guide configured to guide the reflected light "Lr" from the user's retina "Ru" to a separate sensor device 12. Preferably, the measured light "Lr" reflected from the user's retina "Ru" exclusively or predominantly originates from the light of the light source 11 projected onto the user's retina "Ru". In this way, the amount of reflected light can be compared to the amount of projected light. In principle, also environmental light, or light from the display generator 14 could be used, alternatively or in addition to the integrated light source 11.

In some embodiments, e.g. as shown in FIG 2, the HMD system comprises a display generator 14 configured to generate a visible image "Iv", i.e. image comprising or essentially consisting of visible light, e.g. light in a visible wavelength range between 380 - 750 nm, preferably between 450 - 650 nm. In one embodiment, the visible image "Iv" represents and/or depicts informational content. In another or further embodiment, the informational content may include driving or flight parameters of the vehicle, e.g. airplane, its surroundings, user information, and/or general information. In another or further embodiment, the informational content may be represented in the visible image as letters, numbers, other signs, lines, surfaces, overlays, et cetera. In other or further embodiments, a display projection system 15,16 is configured to project the visible image "Iv" for conveying the informational content to the user. In a preferred embodiment, the visible image "Iv" is projected (directly) onto a user's retina "Ru". In another or further embodiment, the visible image "Iv" is projected on a transparent surface visible to the user. In general, the informational content may be conveyed when the user is able to clearly see, register, and interpret the image projected on their retina. Together, the display generator 14 and display projection system 15,16 may have the functionality of a typical head-mounted display 10.

In some embodiments, light source 11 comprises an infrared light source configured to (exclusively or additionally) generate infrared light (that is essentially invisible to the user). For example, the infrared light includes or essentially consists of light in a wavelength range above 700 nm, preferably above 750 nm, e.g. between 800 nm and 10 µm, or higher. In one embodiment, the generated infrared light is directed to illuminate the user's retina "Ru". In another or further embodiment, the infrared light is directed using at least some of the same optical components used for projecting the visible image. In other words, the optical path of the projected HMD image may partially overlap the optical path of the illuminating infrared light and/or the reflected infrared light.

In other or further embodiments, the sensor device 12 is configured to measure part of the generated infrared light "Lr" reflected back from the user's retina "Ru". In one embodiment, the measured part depends at least in part on (variable) absorption and/or reflection and/or scattering of the infrared light in the user's retina "Ru". For example, this may depend on physiological conditions. In another or further embodiment, the computing device 13 is configured to determine the physiological parameter "Pf" of the user based on the absorption and/or reflection and/or scattering of the infrared light in the user's retina "Ru".

In some embodiments, e.g. as shown in FIG 2, the system comprises a beam splitter and/or combiner 17. In one embodiment, the beam splitter and/or combiner 17 is configured to pass (e.g. reflect or transmit) light of the visible image "Iv" generated by the display generator 14 to the user's retina "Ru". In another or further embodiment, the beam splitter and/or combiner 17 is configured to pass (e.g. transmit or reflect) light "Lr" reflected back from the user's retina "Ru" to the sensor device 12.

In one embodiment, the beam splitter and/or combiner 17 comprises a dichroic mirror configured to pass visible light of the visible image generated by the display generator 14 to the user's retina "Ru". In another or further embodiment, the dichroic mirror is configured to pass infrared light between the user's retina "Ru" and the monitoring system (and vice versa). In one embodiment, infrared light generated by the infrared light source 11 is passed to the user's retina "Ru". In another or further embodiment, infrared light reflected back from the user's retina "Ru" is passed to the sensor device 12. Alternatively, or in addition to a dichroic beam splitter, also other types of beam splitters/combiners can be used, e.g. simply splitting a ratio of light and/or dependent on wavelength, polarization, et cetera. Alternatively, or additional to the infrared light source, also light originating from a reflection of visible light, e.g. from the display generator 14, or other light source, may be measured and used for determining one or more physiological parameters.

In some embodiments, the system comprises at least one reflecting window 15, i.e. semi-transparent, or partially transmitting and partially reflecting optical surface. In one embodiment, the reflecting window 15 is connected to (e.g. carried by) the head-mount 20, and configured to be, in use, arranged in front of at least one of user's eyes when the system is worn (in the intended manner) on the user's head "H" by the head-mount 20. In one embodiment, the reflecting window 15 is configured to allow the user to see an external environment E (beyond the HMM system) through the reflecting window 15. Preferably, the user is able to unrestrictedly observe their external environment through the reflecting window 15, e.g. including the environment at a distance of more than one meter from the user, or any distance beyond the HMD system. In another or further embodiment, the reflecting window 15 is configured to reflect light of the light source 11 onto the user's retina "Ru". In another or further embodiment, the reflecting window 15 is configured to reflect light from the user's retina "Ru" back to the sensor device 12.

In some embodiments, the reflecting window 15 comprises glass, plastic, or other (semi) transparent (and partially reflecting) material. For example, this window or material is, in use, arranged in front of at least one of the user's eyes. In one embodiment, the material is configured to allow the user to see through the reflecting window 15. In another or further embodiment, the material is configured to reflect the visible image from the display generator 14, e.g. for projection of the visible image onto the user's retina "Ru". In another or further embodiment, the material is configured to reflect light from the user's retina "Ru", e.g. to the HMM system 10 for measurement by the sensor device 12.

To allow the user to properly see through, the reflecting window 15 is preferably configured to transmit a substantial fraction of visible light, e.g. at least 50%, more preferably at least 80%, most preferably at least 90%. On the other hand, the reflecting window 15 may also be configured to reflect or otherwise filter excessive external light, e.g. glare. So, the light transmission may also be less, depending on circumstances. To sufficiently reflect the visible image "Iv" from the display generator 14 for projection onto the user's retina "Ru", preferably at least an inside of the reflecting window 15 is configured to reflect visible light, e.g. at least 1% in a visible wavelength range and/or at least at a wavelength of the HMD display, preferably at least 5%, at least 10%, or more. Alternatively, or in addition to the reflectivity of the reflecting window 15, also a light intensity of the display generator 14 may be adjusted, e.g. increased when the reflectivity is relatively low. Depending on the wavelength used by the HMM system 10, the reflecting window 15 may also be configured to reflect wavelengths outside the visible spectrum, e.g. infrared. In one embodiment, the light source 11 is an infrared light source, and the reflecting window 15 is configured to reflect (a majority of) infrared light, e.g. while passing (a majority of) visible light. Alternatively, the light source 11 may be a visible light source and the reflecting window 15 may both reflect and transmit respective portions of visible light, e.g. more than 5% each.

In some embodiments, e.g. as illustrated in FIGs 1 and 2, the at least one reflecting window 15 forms one or more curved reflecting surfaces, e.g. configured to image light rays onto the user's retina "Ru" of a respective eye and/or focus light rays through the pupil of the respective eye. For example, the reflecting window 15 acts as a parabolic, spherical, or other shaped mirror, forming part of a projection system to bring source light Ls from the light source 11 to the user's retina "Ru", and/or bring reflected light "Lr" from the user's retina "Ru" to the sensor device 12.

In other or further embodiments (not shown), the HMM may be part of an augmented reality (AR) or virtual reality (VR) system. In one embodiment of an augmented reality system, instead of viewing the external environment E directly via the reflecting window 15, the user may be presented with a (live) recorded image of the external environment which is projected onto the user's retina Ru, e.g. mixed with augmented reality features and/or informational content. For example, the reflecting window 15 can be replaced by a full reflector, or an image projection system with integrated monitoring system can be placed directly in front of the user's eye(s). In one embodiment of a virtual reality system, instead of viewing the external environment E, the user may be presented with a virtual environment which is projected onto the user's retina Ru.

In some embodiments, e.g. as illustrated in FIG 2, the HMD system 100 comprises or couples to the HMM system 10, e.g. the same or similar as described with reference to FIG 1. In one embodiment, the HMM system 10 comprises at least a sensor device 12 configured to measure light "Lr" reflected from the user's retina "Ru". In a preferred embodiment, the HMM system 10 is integrated with the HMD system 100. For example, the sensor device 12 is configured to measure light "Lr" reflected from the user's retina "Ru" using at least part of the display projection system 15,16. Typically, the display projection system comprises one or more optical components 16,18 such lenses and/or (curved) mirrors to guide and/or project the visible image "Iv" generated by the display generator 14 onto the user's retina "Ru". As shown, also the (curved) reflecting window 15 can be used as part of the display projection system. To project a sharp image onto the retina, a focal plane of the projection system may be configured and/or adaptable to take into account a focal distance of the lens which is already present as part of the user's eye. For example, the projection system may project the image with an apparent object distance at infinity and/or adapted to the typical and/or variable focal distance of the user's eye. Accordingly, the user's eyes do not need to refocus to view the information. In a preferred embodiment, the measured light "Lr" reflected from the user's retina "Ru" is guided by, or passed through, at least some of the same optical components as used for projecting the visible image "Iv".

In some embodiments, the system comprises a computing device 13 configured to determine a physiological parameter "Pf" of the user, e.g. based at least in part on the measured light "Lr" reflected from the user's retina "Ru". Also other or further sensor measurements may be included to determine the parameter "Pf". In one embodiment, the computing device 13 comprises a processor and/or memory with instructions configured to perform operational acts including processing of sensor data, calculation of one or more parameters based on the processing, and/or outputting the one or more calculated parameters.

In some embodiments, the computing device 13 is configured to determine, e.g. continuously monitor, a physiological parameter "Pf" of the user based on the measured light "Lr". It will be appreciated that the present disclosed systems and methods allow continuously monitoring a user's physiological condition while minimally restricting the user's activities such as operating a vehicle. For example, the HMM system may be configured to monitor one or more physiological parameters "Pf" continuously, or at (regular) intervals, e.g. monitor for at least one minute, at least five minutes, at least ten minutes, or more, e.g. indefinitely until the device is stopped or removed from the user's head. Of course, this is significantly different from a traditional ophtalmoscope which is generally a non-wearable device and aimed at determining deterioration of the eye, not continuous monitoring of physiological parameters as described herein.

As described herein, the system may take measurements of the user's retina "Ru" which is located inside a respective eye of the user. In some embodiments, the system may rely on measurement of a single eye. In other or further embodiments, the system may use simultaneous or consecutive measurements on both eyes. So, it will be understood that parts of the system may be duplicated and/or or split into separate path ways, to illuminate both eyes and/or measure reflection of both eyes. In some embodiments, even when measuring both eyes, some parts of the system can be combined. For example, a single light source 11 and/or single sensor device 12 can be used by splitting the light paths in a projection system. In other or further embodiments, also separate light sources and/or separate sensor devices can be used, or even two completely separate optical systems. Preferably at least the same computing device 13 is used for processing both measurements and determining a physiological parameter "Pf" based on both measurements.

In some embodiments, e.g. as shown in FIG 2, the HMD system 100 as described herein may be part of a helmet or similar head-worn device. In one embodiment, the helmet includes (contains and/or houses) one or more, preferably all of the display generator 14, display projection system 15,16, as well as the HMM system 10 integrated therewith. In another or further embodiment, an audio or haptic feedback signal indicating the physiological parameter "Pf" is output by the helmet, e.g. warning signal provided via an earpiece, or vibration of the helmet to alert the user. A feedback device can also be provided in other types of head-worn devices, such as the spectacle design of FIG 1.

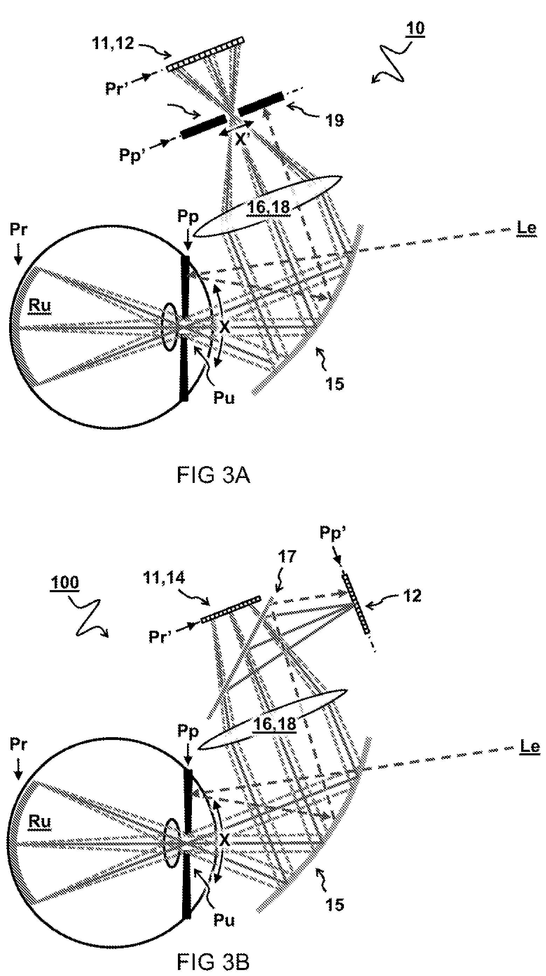

FIGs 3A and 3B illustrate additional features for isolating relevant light originating from the user's retina "Ru" and/or filtering out background light. FIG 3A illustrated one embodiment implemented in an HMM system 10. FIG 3B illustrates another embodiment implemented in an HMD system 100 comprising a display generator 14 in addition to the HMM system 10. It will be understood that the features shown in FIG 3A could also be implemented in FIG 3B, and vice versa. For example, the spatial filter 19 could also be implemented in an HMD system. Also, it will be understood that the features of either FIGS 3A and 3B can be implemented in FIGs 1 and/or FIG 2, or vice versa.

According to theinvention, the system (HMM or HMD) as described herein comprises a projection system 16,18 configured to project at least some of the source light Ls of the light source 11 onto the user's retina "Ru" via a user's pupil "Pu" arranged at a first pupil plane "Pp" of the projection system 16,18 and/or project at least some of the reflected light "Lr" from the user's retina "Ru" onto the sensor device 12. It will be understood that the user's pupil "Pu" refers to the respective pupil of the user's eye corresponding to the respective user's retina "Ru" to be measured. So, the present teachings could be implemented for one eye, or repeated for both eyes.

As shown, in FIGs 3A and 3B, the user's pupil "Pu" can have a variable position "X" which may affect the measurement of reflected light "Lr". In some embodiments, the system simply measures all reflected light which may include signal light originating from the user's retina "Ru" through the pupil as well as background light, e.g. originating from reflection of the surrounding iris. For example, the background light may originate from external light "Le" entering the system, or light originating from the system, e.g. light source 11, but being reflected off other parts of the eye, e.g. iris. In one embodiment, the computing device 13 may be capable of isolating the relevant signal(s) from background signal(s). In another or further embodiment, the user may be presented with a visible image to direct the eye in a specific direction. This may alleviate the amount of background signal.

According to the invention, the system as described herein comprises pupil tracking means. In one embodiment, the pupil tracking means comprises a devices or components such as a spatial filter 19, which may have a fixed or adjustable position. In another or further embodiment, at least some of the functionality of the pupil tracking means is implemented in the already described devices, such as the light source 11 and/or the sensor device 12 and/or the computing device 13.

According to the invention, the pupil tracking means is configured to determine an image position X' of the user's pupil "Pu" in a second pupil plane "Pp"' of the projection system 16,18, conjugate to the first pupil plane "Pp". In other or further embodiments, the pupil tracking means is configured to use reflected light "Lr" exclusively originating from the position X of the user's pupil "Pu" for measuring the user's retina "Ru", while filtering out light reflected off surrounding parts of the user's eye. As will be understood, this may improve sensitivity of the measurement. For example, of all source light Ls reflected from the user's eye, part of the reflected light coinciding with the determined image position X' in the second pupil plane "Pp"' can be isolated, wherein the isolated part of the reflected light corresponds to the reflected light "Lr" exclusively originating from a position X of the user's pupil "Pu". Accordingly, it is possible to exclusively use reflected light "Lr" originating from the position X of the user's pupil "Pu" for measuring the user's retina "Ru". The intended light originating from the retina can be isolated and/or other light, e.g. originating from other reflections can be filtered.

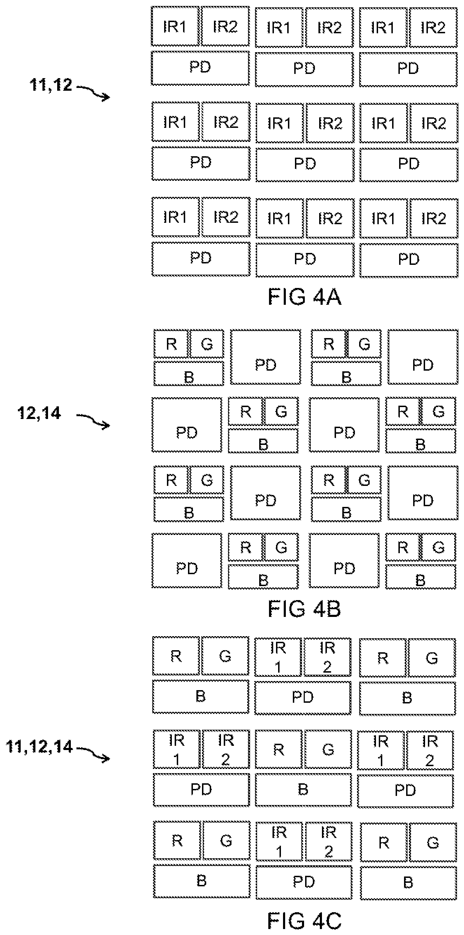

According to the invention, e.g. as shown in FIG 3A, the filtering comprises spatially filtering light passing the second pupil plane "Pp"' from the light source 11 to the user's retina "Ru", and/or from the user's retina "Ru" to the sensor device 12, to exclusively pass light coinciding with the determined image position X' of the user's pupil "Pu". For example, a position X' of the hole through the spatial filter 19 can be adjusted based on the determined position X of the user's pupil "Pu". In another or further embodiment, e.g. as shown in FIG 3B, the light originating from the user's pupil "Pu" is isolated providing a sensor device 12 at the second pupil plane "Pp"' to exclusively measure the reflected light "Lr" at the determined image position X' of the user's pupil "Pu". For example, a position X' of where light is measured by the sensor device 12 (or the position of the sensor device 12, e.g. having one sensor element) can be adjusted based on the determined position X of the user's pupil "Pu". Of course it can also be envisaged to isolate the respective signal by the computing device 13 using only a subset of measurement from the sensor device 12 corresponding to the image position X' of the user's pupil "Pu". In another or further embodiment (not shown), a light source 11 is provided at the second pupil plane Pp' (or any pupil plane conjugate to the first pupil plane Pp) to exclusively generate the source light Ls at the determined image position X' of the user's pupil "Pu". For example, a position X' of where light is generated by the light source 11 (or the position of the light source 11) can be adjusted based on the determined position X of the user's pupil "Pu". This may be similar to filtering the source light Ls using a spatial filter 10 as shown in FIG 3A. Also combinations are possible. For example, a combined light source 11 and sensor device 12 with respective light generating and/or sensing elements, e.g. as shown in FIGs 4A and 4C, can be placed at the second pupil plane Pp' alternative to, or instead of the spatial filter 19 as shown in FIG 3A.

In one embodiment, the sensor device 12 may act as an imaging device for detecting the position of the pupil. In another or further embodiment, a separate pupil detector, e.g. camera, can be used to detect the pupil position. In one embodiment, the pupil position is detected by imaging the user's eye and determining the relative position of the pupil in the image. In another or further embodiment, the pupil position is determined by the amount of light reflected onto a detector, e.g. the sensor device 12. For example, the pupil position may appear on the detector as a dark spot, with relatively low (reflected) light intensity compared to the surrounding iris or sclera.

In some embodiments, e.g. as shown in FIG 3A, the system comprises a spatial filter 19 arranged in the conjugate pupil plane "Pp"' and comprising a pinhole, e.g. forming an entrance or exit pupil of the optical system, wherein a position of the pinhole is controlled to exclusively pass the reflected light "Lr" originating from the variable pupil position X and/or exclusively pass the source light Ls projected onto the variable pupil position X. In other or further embodiments, e.g. as shown in FIG 3B, the sensor device 12 comprises a sensor array with pixels arranged in the conjugate pupil plane "Pp"'. For example, the sensor array is controlled to exclusively measure the reflected light "Lr" originating from the variable pupil position X, or at least capable of filtering the other light. Similarly, the light source 11 may comprise a light source array arranged in the conjugate pupil plane "Pp"' and controlled to exclusively generate the source light Ls being projected onto the variable pupil position X.

In some embodiments, e.g. as shown in FIG 3A, the light source 11 and/or sensor device 12 is arranged at an object plane or image plane Pr' which is conjugate with the image or object plane Pr of the user's retina "Ru". For example, this allows to project a specific image onto the user's retina "Ru" and/or measure an image of the user's retina "Ru". In other or further embodiments, e.g. as shown in FIG 3B, the light source 11 and/or display generator 14 is arranged at an object plane or image plane Pr' which is conjugate with the image or object plane Pr of the user's retina "Ru". In other or further embodiments, e.g. as shown in FIG 3B, the sensor device 12 is arranged at a second pupil plane Pp' which is conjugate with the pupil plane Pp of the user's pupil Pu. This allows isolating light on the sensor device 12 corresponding to light originating from the pupil. Alternatively, or additionally, also the light source could be placed at a specific position in a pupil plane to exclusively illuminate the pupil.

FIGs 4A-4C illustrate integrated devices for generation and measuring of infrared and/or visible light. In some embodiments, e.g. as shown in FIG 4A, the light source 11 and sensor device 12 can be combined, e.g. on a single substrate or foil. In one embodiment, the substrate comprises one, two, or more different light source elements to generate light at one two, or more different wavelengths. For example, in the embodiment of FIG 4A, two different infrared sources IR1,IR2 are shown. In another or further embodiment, the substrate comprises one, two, or more different light sensing elements to sense light at one two, or more different wavelengths (e.g. using respective wavelength filters), or any wavelength. For example, in the embodiment of FIG 4A, one type of photodiode element PD is shown interlaced between the light source elements IR1,IR2. In other or further embodiments, e.g. as shown in FIG 4B, the sensor device 12 and display generator 14 can be combined, e.g. on a single substrate or foil. For example, the substrate comprises light generating elements such as OLEDs or microLEDs, to generate light at different colors (e.g. R,G,B), interlaced with light sensing elements such as photodiodes (PD). In other or further embodiments, e.g. as shown in FIG 4C, each of the light source 11, sensor device 12, and display generator 14 can be combined, e.g. on a single substrate or foil. In other or further embodiments (not shown) the light source 11 (e.g. comprising IR light sources) and display generator 14 (e.g. comprising visible light sources) can be combined on a single substrate or foil. Of course the elements described in any of the shown or described embodiments could also be placed on multiple substrates, e.g. with transparent portions of the front substrate passing light to or from a back substrate. The different substrates or elements could also be placed at different positions, e.g. corresponding to an object or image plane Pr' which is conjugate with the image or object plane Pr of the user's retina "Ru", as illustrated in FIGs 3A and 3B.

In some embodiments, the system (HMM or HMD) as described herein may be part of, or communicatively couple with, a control system (not shown) for controlling a vehicle (not shown), e.g. airplane. In one embodiment, the control system comprises the HMM system 10 and/or HMD system 100, e.g. helmet, as described herein configured to determine a physiological parameter "Pf" of a user operating the vehicle. In another or further embodiment, the control system comprises a controller (not shown) configured to receive an indication of the determined physiological parameter "Pf" and adapt control of a vehicle based on the received indication. For example, the controller may receive an indication of one or more of a low oxygenation of blood, low pulse, irregular eye movement, et cetera. This may indicated the user (driver, pilot) is impaired. For example, the controller may enable (or disable) partial or full automatic control of the vehicle based on the received indication.

It will be understood, that aspects as described herein with reference to various systems and devices, can also be embodied in corresponding method, and vice versa. Accordingly, in some aspects, the present disclosure also provides a method of monitoring, or generally determining, a physiological parameter "Pf" using a head-mounted monitoring (HMM) system 10. The monitoring can take place while the HMM system 10 is worn on a user's head "H", e.g. with a part of the HMM system arranged in front of the user's eye(s), while allowing the user to freely view their environment through said part and/or view a visible image representing informational content projected via said part onto the user's retina. In one embodiment, the method comprises projecting source light onto the user's retina via said part of the HMM system arranged in front of the user's eye(s), measuring light reflected from the user's retina, and determining a physiological parameter of the user based on the measured light. For example, a physiological condition of the user can be monitored by continuously and/or intermittently comparing the physiological parameter to a reference value or range. For example, the monitoring may include determining a healthy or impaired physiological condition, optionally taking further action based on the monitoring, e.g. adapt control of a vehicle.

In other or further aspects, the present disclosure provides a method of monitoring a physiological parameter "Pf" using an HMM system 10 integrated with an HMD system 100. In one embodiment, the method comprises generating a visible image "Iv" representing informational content and projecting the visible image "Iv". In another or further embodiment, the method comprises using a display projection system 15,16, onto a user's retina "Ru" for conveying the informational content to the user. In another or further embodiment, the method comprises measuring light "Lr" reflected from the user's retina "Ru" using at least part of the display projection system 15,16. For example, the reflecting window 15 can be used both for projecting the visible image "Iv" onto the user's retina Ru, as for reflecting back light from the user's retina Ru to the sensor device 12. Also other or further parts of the projection system, such as one or more lenses, mirrors, filters, et cetera, can be shared between the display projection system of the HMD and projection optics of the HMM. See, for example, the shared lens 16,18 in FIGs 3A and 3B. In another or further embodiment, the method comprises determining the physiological parameter "Pf" of the user based on the measured light "Lr". Some or all of these steps may also be embodied as a non-transitory computer-readable medium storing instructions that, when executed by one or more processors, cause a device to perform the one or more steps, e.g. in combination with a suitable HMM and/or HMD system as described herein.

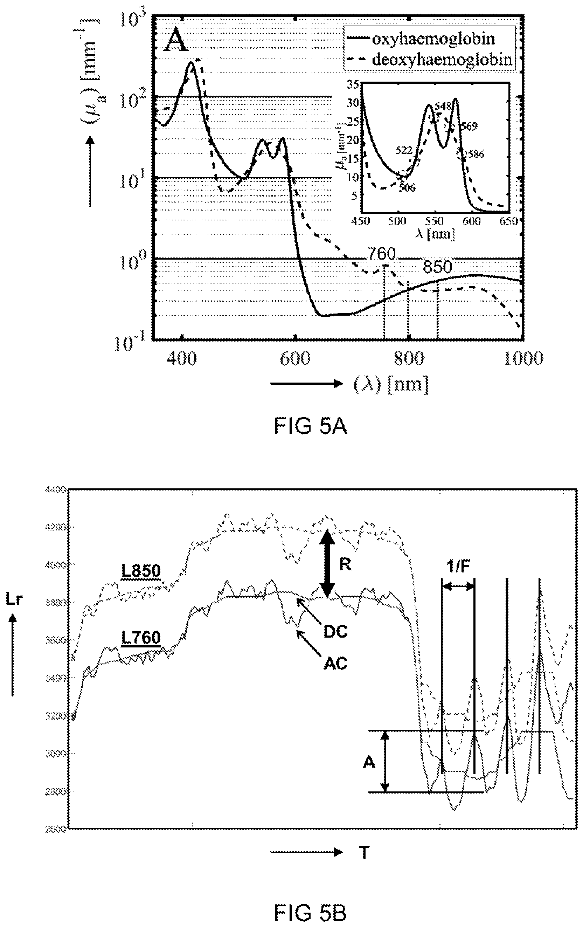

FIG 5A illustrates absorption spectra of oxyhaemoglobine and deoxyhaemoglobine; FIG 5B illustrates a measurement of reflected light "Lr" as function of time, and at different wavelengths (here: 760 nm and 850 nm which is also indicated in FIG 5A).

In some embodiments, the system as described herein is configured to measure the reflected light "Lr" as function of time "T". For example, the sensor device is configured to take periodic measurements of respective light intensity. Preferably, the system (e.g. sensor device and computing device) is capable of measuring and/or processing at least one measurement per second, more preferably at least five measurements per second, most preferably at least ten measurements per second, e.g. up to one hundred measurements per second, or more. Providing relatively quick and/or many measurements, may allow to adapt to changing conditions, such as eye movement. For example, each measurement may take less than ten milliseconds, less than five milliseconds, or even less than one millisecond. Furthermore, providing more measurements per second may allow to better monitor also physiological parameters characterized by a change or frequency.

The variation of the measured signal may also be referred to as the AC signal, while the baseline or DC signal may be relatively static, at least more slowly varying than the AC signal. For example, the DC signal may be determined as a moving average while AC signal may determine by subtracting the DC signal or using another average (e.g. less points). Typically, frequencies in the signal may be filtered to isolate a frequency range which may physically correspond to heart rate or other physiological parameter of interest, e.g. breathing; and/or isolate a more slowly changing DC signal. Also other or further filtering may be applied, e.g. isolating or compensating for changes in baseline which may be attributed to eye movement, breathing, head movement, et cetera.

In one embodiment, the sensor device 12 is configured to measure a light intensity of the reflected light "Lr" as function of time "T". In another or further embodiment, the computing device 13 is configured to calculate a frequency "F" of a variation of the light intensity for determining a physiological parameter "Pf" indicating a heart rate of the user. For example, an elevated or lowered heart rate may be indicative of various physiological conditions, such as stress and/or physical strain. In another or further embodiment, the computing device 13 is configured to calculate a variation of the light intensity for determining a physiological parameter "Pf" indicating a (cerebral) blood pressure. For example, an amplitude "A" of the variation of the light intensity, as shown, may be an indication of blood pulsation amplitude, which may be correlated with blood pressure. For example, an AC component of the signal may be used as indication how much blood is still arriving at the retina, which may be affected by G-Force.

Also overall (DC) variation of the light intensity may be used to derive physiological information, such as blood pressure. In one embodiment, short time variations may be correlated with heart rate rhythm. In another or further embodiment, long term variations may be correlated with changes in blood pressure, e.g. as a result of stress, exertion, et cetera. In another or further embodiment, a shape of the signal may be used. For example, when the blood pressure is relatively high, this may be indicated by a flattening of the peak and/or low value, e.g. due to reaching a maximum stretchability of the blood vessel. In some embodiments, the (varying) light intensity signal may be combined with a separate (timed) ECG signal, e.g. to compare the time of arrival. For example, for a relatively high blood pressure, the arrival time may be relatively shorter than for a relatively low blood pressure. In one embodiment, the monitoring system as described herein comprises or couples to a separate ECG sensor, e.g. measuring the heart rate at another part of the user's body (not via the retina).

In some embodiments, the system (HMM or HMD) is configured to measure the reflected light "Lr" as function of wavelength λ. For example, reflected light may be spectrally resolved and/or filtered, to isolate or separately measure one, two, three, or more wavelengths in a visible or infrared region of interest. In one embodiment, one or more sensor elements of the sensor device 12 comprises a respective spectral band filter to measure light at a specific wavelength. Alternatively, or in addition to filtering/resolving the reflected light, the wavelength of the light source 11 may be modulated. For example, the wavelength of source light may be modulated between distinct wavelengths while measuring the respective intensity of reflected light. Preferably, the measures wavelengths include one or more wavelengths that can be used to distinguish absorption in blood vessels of the retina versus other retinal tissue. In this way, various physiological parameters related to blood supply, or absence thereof, may be monitored.

In some embodiments, the sensor device 12 comprises an imaging device configured to generate an image of the user's retina "Ru". In one embodiment, the computing device 13 is configured to process the image of the user's retina "Ru" to determine a position of at least one blood vessel in the retina (e.g. retinal artery), and to determine the physiological parameter "Pf" of the user based on the measured light "Lr" at the position of at least one blood vessel. In another or further embodiment, the computing device 13 is configured to process the image of the user's retina "Ru" to determine a position of background retinal tissue without blood vessel and determine the physiological parameter "Pf" of the user based on the measured light "Lr" at the position of the background retinal tissue. Alternatively, the retinal tissue does not need to be imaged. For example, the reflected light "Lr" can be combined or (non-imaged) light from the user's retina "Ru" can be collected and/or used to determine the physiological parameter "Pf". In another or further embodiment, the computing device 13 is configured to determine the physiological parameter "Pf" of the user based on a difference between the measured light "Lr" at the position of the at least one blood vessel and the measured light "Lr" at the position of the background retinal tissue. By distinguishing light originating from a blood vessel versus background retinal tissue, sensitivity can be improved. Alternatively, such distinction on the sensor side may not be necessary e.g. if the light exclusively or predominantly illuminates a blood vessel. For example, the HMD system may already be configured to track eye movement and adjust the light source 11 accordingly.

In other or further embodiments, light reflected off the whole retina, or an (non-distinguished) portion thereof, may be collected and used as basis for determining at least one physiological parameter "Pf" (without distinguishing blood vessels). For example, the measurement device may be sufficiently sensitive to distinguish small variations even on top of a larger background signal. In one embodiment, the measurement device is configured to collect light exclusively reflected off the retina, measure the collected light, and determine at least one physiological parameter "Pf" based on a variation in the measured light exclusively reflected off the retina. As will be appreciated, sensitivity of the measurement device may be improved by using reflected light exclusively or predominantly originating from the position of the user's pupil. For example, pupil tracking means can be used to dynamically filter light originating from locations outside the position of the user's pupil as described herein, e.g. with reference to FIGs 3A and 3B.

In general, it will be understood that the sensor device 12 may comprise one or more of a single light detecting element, multiple light detecting elements (e.g. two detectors and/or a pixel array configured to measure multiple spectral features of the reflected light), a single imaging device, and/or multiple imaging devices (e.g. one for each wavelength). In one embodiment, a single light detecting element can be used to measure a signal corresponding to the collected light exclusively originating from the retina, or the measurement of multiple light detecting elements measuring light exclusively originating from the retina can be combined into one signal. Accordingly, the collected signal can be used to determine at least one physiological parameter "Pf", e.g. based on a variation in the signal.

In some embodiments, the computing device 13 comprises a neural network or machine learning algorithm (A.I.) to determine one or more physiological parameters "Pf" based on the reflected light "Lr". In one embodiment, the neural network is trained to correlate measured light reflection with a value for one or more physiological parameters "Pf". For example, during a training of the neural network, the one or more physiological parameters are measured independently and used as feedback in the learning. Preferably, the neural network is trained for a specific user. For example, the network may be able to distinguish the particular characteristics of the user's eye (blood vessels) and/or corresponding physiological condition. In one embodiment, the neural network is provided with one or more images of the reflected light representing the retina and outputs an indication for at least one physiological parameters. Preferably, the neural network is provided with multiple images at different instances of time and/or at different wavelengths. In this way, a more informed output may be calculated.

In some embodiments, the system (HMM or HMD) comprises a feedback device (not shown) configured to output a feedback signal based on the determined physiological parameter "Pf". In one embodiment, the feedback may be provided in one or more of a visual, audio, or haptic feedback signal. For example, the feedback signal may alert the user or other personnel of measuring a nominal value or deviation from a nominal value. In one embodiment, a visual signal indicating the physiological parameter "Pf" is provided via the display generator 14. Alternatively or additionally, an electric (or optical) signal may be provided, e.g. as feedback for other systems or devices.

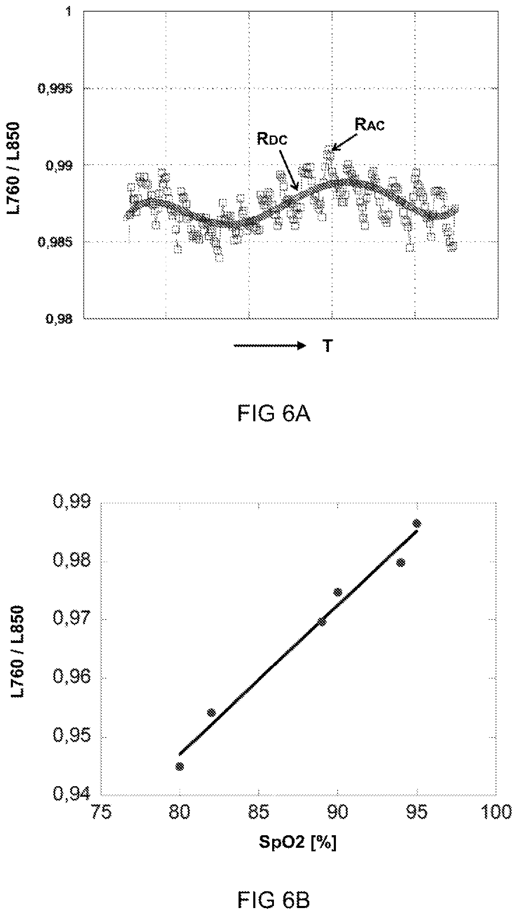

FIG 6A illustrates a ratio of light intensity measured at different wavelengths. The figure shows both the ratio RAC of the raw (alternating) signals L760/L850, and the ratio of the corresponding DC signals (moving average); FIG 3B illustrates a correlation between the ratio of light intensity and oxygenation level measured by an independent sensor.

In some embodiments, the sensor device 12 is configured to measure a respective light intensity (L850,L760) of the reflected light "Lr" at (at least) two distinct wavelengths λ, e.g. here 760 nm and 850 nm, but this could of course also be measured at other or further suitable wavelengths. In one embodiment, the computing device 13 is configured to determine a physiological parameter "Pf" including an indicative value of (cerebral) oxygenation based on the respective light intensity at the two distinct wavelengths λ. This is also known as a retinal oxygenation measurement. For example, the indicative value of oxygenation is based on a ratio and/or difference between the respective light intensity at two distinct wavelengths λ. For example, FIG 6A shows the ratio (L760 / L850) of signals such as shown in FIG 5B. Also more than two wavelengths may be used. Preferably, the wavelengths include an anisosbestic wavelength where the absorption coefficient of oxyhaemoglobine and deoxyhaemoglobine are different. More preferably at least two anisosbestic wavelengths are used, most preferably having a different, e.g. opposite ratio. For example, at a first measured wavelength (e.g. 760 nm) the absorption of deoxyhaemoglobine may be larger than that of oxyhaemoglobine and at a second wavelength (e.g. 850 nm) it may be smaller.

In some embodiments, the measured one or more wavelengths include an isosbestic wavelength where the absorption coefficient of oxyhaemoglobine and deoxyhaemoglobine are the same, e.g. around 800 nm which is advantageously outside the visible spectrum. The measurement at an isosbestic wavelength may be advantageous as a reference and/or advantageous for measuring other physiological parameters, independent of oxygenation. For example, a single anisosbestic wavelength may be monitored to determine heart rate, blood pressure, et cetera.

For the purpose of clarity and a concise description, features are described herein as part of the same or separate embodiments, however, it will be appreciated that the scope of the invention may include embodiments having combinations of all or some of the features described. For example, while embodiments were shown for a specific HMD and monitoring system, also alternative ways may be envisaged by those skilled in the art having the benefit of the present disclosure for achieving a similar function and result. E.g. optical components may be combined or split up into one or more alternative components. The various elements of the embodiments as discussed and shown offer certain advantages, such as inflight measurement of physiological conditions. Of course, it is to be appreciated that any one of the above embodiments or processes may be combined with one or more other embodiments or processes to provide even further improvements in finding and matching designs and advantages. It is appreciated that this disclosure offers particular advantages to aviation, and in general can be applied for any application of HMD systems. For example, although HMDs were initially developed for military aviation, HMDs are now also used in commercial aircraft, automobiles, and other applications. So the present teachings may also be extended to other fields of use.

The extent of the protection conferred by this patent or application shall be determined by the claims. Nevertheless, the description and drawings shall be used to interpret the claims.

In interpreting the claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise.

## Claims

1. A head-mounted monitoring, HMM, system (10) comprising
a head-mount (20) configured to attach the HMM system (10) to a user's head (H);
a light source (11) configured to project source light (Ls) onto a user's retina (Ru) when the HMM system is attached to the user's head (H) by the head-mount (20);
a sensor device (12) configured to measure reflected light (Lr) reflected from the user's retina (Ru);
a projection system (16,18) configured to project source light (Ls) of the light source (11) onto a user's eye, with at least some of the source light (Ls) reaching the user's retina (Ru), inside the eye, via a user's pupil (Pu), of the eye, wherein the user's pupil (Pu) is arranged at a first pupil plane (Pp) of the projection system (16,18);
pupil tracking means configured to
determine an image position (X') of the user's pupil (Pu) in a second pupil plane (Pp') of the projection system (16,18), conjugate to the first pupil plane (Pp), and
use reflected light (Lr) exclusively originating from the position (X) of the user's pupil (Pu) for measuring absorption and/or scattering of the source light (Ls) from the user's retina (Ru), while omitting or filtering out light reflected off surrounding parts of the user's eye, by at least one of
spatially filtering light passing the second pupil plane (Pp') from the light source (11) to the user's retina (Ru), and/or from the user's retina (Ru) to the sensor device (12), to exclusively pass light coinciding with the determined image position (X') of the user's pupil (Pu),
providing a light source (11) at the second pupil plane (Pp') to exclusively generate the source light (Ls) at the determined image position (X') of the user's pupil (Pu),
providing a sensor device (12) at the second pupil plane (Pp') to exclusively measure the reflected light (Lr) at the determined image position (X') of the user's pupil (Pu); and
a computing device (13) configured to continuously monitor a physiological parameter (Pf) of the user based on the measured light (Lr).

2. The HMM system (10) according to claim 1,
wherein the light source (11) comprises an infrared light source configured to generate infrared light illuminating the user's retina (Ru);
wherein the sensor device (12) is configured to measure part of the generated infrared light (Lr) reflected back from the user's retina (Ru) depending at least in part on absorption and/or scattering of the infrared light in the user's retina (Ru);
wherein the computing device (13) is configured to determine the physiological parameter (Pf) of the user based on the absorption and/or scattering of the infrared light in the user's retina (Ru).

3. The HMM system (100) according to any of the preceding claims, comprising at least one reflecting window (15), connected to the head-mount (20), and configured to be, in use, arranged in front of at least one of user's eyes when the HMM system (10) is worn on the user's head (H), wherein the reflecting window (15) is configured to
allow the user to see an external environment (E) through the reflecting window (15);
reflect light of the light source (11) onto the user's retina (Ru); and
reflect light from the user's retina (Ru) back to the sensor device (12).

4. The HMM system (10) according to any of the preceding claims, wherein the sensor device (12) is configured to measure a respective light intensity (L850,L760) of the reflected light (Lr) at at least two distinct wavelengths (λ), wherein the computing device (13) is configured to determine a physiological parameter (Pf) including an indicative value of oxygenation based on comparing the respective light intensity (L850,L760) at the at least two distinct wavelengths (λ).

5. The HMM system (10) according to any of the preceding claims, wherein the sensor device (12) is configured to measure a light intensity of the reflected light (Lr) as function of time (T), wherein the computing device (13) is configured to calculate a physiological parameter (Pf) indicating a blood pressure of the user based on a variation (A) of the light intensity.

6. The HMM system (10) according to any of the preceding claims, wherein the sensor device (12) is configured to measure a light intensity of the reflected light (Lr) as function of time (T), wherein the computing device (13) is configured to calculate a frequency (F) of a variation of the light intensity for determining a physiological parameter (Pf) indicating a heart rate of the user.

7. The HMM system (10) according to any of the preceding claims, wherein the computing device (13) comprises a neural network or machine learning algorithm to determine one or more physiological parameters (Pf) based on the reflected light (Lr).

8. The HMM system (10) according to any of the preceding claims, comprising a feedback device configured to output a feedback signal based on the determined physiological parameter (Pf).

9. A head-mounted display, HMD, system (100) comprising
the HMM system (10) according to any of the preceding claims;
a display generator (14) configured to generate a visible image (Iv) representing informational content; and
a display projection system (15,16) configured to project the visible image (Iv) onto the user's retina (Ru) for conveying the informational content to the user;
wherein the sensor device (12) is configured to measure light (Lr) reflected from the user's retina (Ru) using at least part (15) of the display projection system (15,16).

10. The HMD system (100) according to the preceding claim, comprising a beam splitter/combiner (17) configured to
pass light of the visible image (Iv) generated by the display generator (5) to the user's retina (Ru); and
pass light (Lr) reflected back from the user's retina (Ru) to the sensor device (12).

11. The HMD system (100) according to the preceding claim, wherein the beam splitter/combiner (17) comprises a dichroic mirror configured to
pass visible light of the visible image generated by the display generator (5) to the user's retina (Ru);
pass infrared light generated by the light source (11), comprising an infrared light source, to the user's retina (Ru);
pass infrared light reflected back from the user's retina (Ru) to the sensor device (12).

12. The HMD system (100) according to any of the preceding claims, wherein the display projection system (15,16) comprise a reflecting window (15) configured to
allow the user to see through the reflecting window (15);
reflect the visible image (Iv) from the display generator (14) for projection of the visible image onto the user's retina (Ru); and
reflect light from the user's retina (Ru) to the monitoring system (10) for measurement by the sensor device (12).

13. A control system for controlling a vehicle, the control system comprising
the HMM system (10) or HMD system (100) according to any of the preceding claims configured to determine a physiological parameter (Pf) of a user operating the vehicle; and
a controller configured to receive an indication of the determined physiological parameter (Pf) and adapt control of the vehicle based on the received indication.

14. A method of monitoring a physiological parameter (Pf) using a head-mounted monitoring, HMM, system (10) according to any of claims 1 - 8, while the HMM system is worn on a user's head (H), with a partially reflecting window (15) of the HMM system arranged in front of the user's eyes, while allowing the user to freely view their environment through said partially reflecting window (15) and/or view a visible image (Iv) representing informational content projected via said partially reflecting window (15) onto the user's retina (Ru) inside the user's eye, the method comprising
projecting, by a projection system (16,18), source light (Ls), from a light source (11) onto the user's retina (Ru), via said partially reflecting window (15) of the HMM system arranged in front of the user's eye, and via a user's pupil (Pu) of the user's eye, wherein the user's pupil (Pu) is arranged at a first pupil plane (Pp) of the projection system (16,18);
determining, by pupil tracking means, an image position (X') of the user's pupil (Pu) in a second pupil plane (Pp') of the projection system (16, 18), which is conjugate to the first pupil plane (Pp);
measuring, by a sensor device (12), light (Lr) reflected from the user's retina (Ru);
determining a physiological parameter (Pf) of the user based on the measured light (Lr);
wherein the measured light (Lr) used for the determining of the physiological parameter (Pf) exclusively originates from the position (X) of the user's pupil (Pu), while light reflected off surrounding parts of the user's eye is omitted or filtered out, by at least one of:
spatially filtering light passing the second pupil plane (Pp') from the light source (11) to the user's retina (Ru), and/or from the user's retina (Ru) to the sensor device (12), to exclusively pass light coinciding with the determined image position (X') of the user's pupil (Pu),
exclusively generating the source light (Ls) at the determined image position (X') of the user's pupil (Pu) using the light source (11) provided at the second pupil plane (Pp'),
exclusively measuring the reflected light (Lr) at the determined image position (X') of the user's pupil (Pu) using the sensor device (12) provided at the second pupil plane (Pp').

## Patentansprüche

1. Kopfmontiertes Überwachungssystem, HMM-System, (10), umfassend:
eine Kopfmontage (20), die dazu konfiguriert ist, das HMM-System (10) am Kopf (H) eines Benutzers anzubringen;
eine Lichtquelle (11), die dazu konfiguriert ist, Quelllicht (Ls) auf die Netzhaut (Ru) eines Benutzers zu projizieren, wenn das HMM-System durch die Kopfmontage am Kopf (H) des Benutzers (20) angebracht ist;
eine Sensorvorrichtung (12), die dazu konfiguriert ist, reflektiertes Licht (Lr), das von der Netzhaut (Ru) eines Benutzers reflektiert wird, zu messen;
ein Projektionssystem (16, 18), das dazu konfiguriert ist, Quelllicht (Ls) der Lichtquelle (11) auf das Auge eines Benutzers zu projizieren, wobei mindestens etwas des Quelllichts (Ls) die Netzhaut (Ru) des Benutzers innerhalb des Auges über die Pupille (Pu) eines Benutzers des Auges erreicht, wobei die Pupille (Pu) des Benutzers an einer ersten Pupillenebene (Pp) des Projektionssystems (16, 18) eingerichtet ist;
Pupillenverfolgungsmittel, dazu konfiguriert, um
eine Bildposition (X') der Pupille (Pu) des Benutzers in einer zweiten Pupillenebene (Pp') des Projektionssystems (16, 18), die zu der ersten Pupillenebene (Pp) konjugiert ist, zu bestimmen und
ausschließlich reflektiertes Licht (Lr), das von der Position (X) der Pupille (Pu) des Benutzers stammt, zur Messung von Absorption und/oder Streuung des Quelllichts (Ls) an der Netzhaut (Ru) des Benutzers zu verwenden, wobei Licht, das von umgebenden Teilen des Auges des Benutzers reflektiert wird, weggelassen oder herausgefiltert wird durch mindestens eines von räumlichem Filtern von Licht, das die zweite Pupillenebene (Pp') von der Lichtquelle (11) zu der Netzhaut (Ru) des Benutzers und/oder von der Netzhaut (Ru) des Benutzers zu der Sensorvorrichtung (12) durchläuft, um ausschließlich Licht durchzulassen, das mit der bestimmten Bildposition (X') der Pupille (Pu) des Benutzers zusammenfällt,
Bereitstellen einer Lichtquelle (11) an der zweiten Pupillenebene (Pp'), um ausschließlich das Quelllicht (Ls) an der bestimmten Bildposition (X') der Pupille (Pu) des Benutzers zu erzeugen,
Bereitstellen einer Sensorvorrichtung (12) an der zweiten Pupillenebene (Pp'), um ausschließlich das reflektierte Licht (Lr) an der bestimmten Bildposition (X') der Pupille (Pu) des Benutzers zu messen; und
eine Rechenvorrichtung (13), die dazu konfiguriert ist, kontinuierlich einen physiologischen Parameter (Pf) des Benutzers basierend auf dem gemessenen Licht (Lr) zu überwachen.

2. HMM-System (10) nach Anspruch 1,
wobei die Lichtquelle (11) eine Infrarotlichtquelle aufweist, die dazu konfiguriert ist, Infrarotlicht, das die Netzhaut (Ru) des Benutzers beleuchtet, zu erzeugen;
wobei die Sensorvorrichtung (12) dazu konfiguriert ist, einen Teil des erzeugten Infrarotlichts (Lr), das von der Netzhaut (Ru) des Benutzers reflektiert wird, abhängig zumindest zum Teil von einer Absorption und/oder Streuung des Infrarotlichts in der Netzhaut (Ru) des Benutzers, zu messen;
wobei die Rechenvorrichtung (13) dazu konfiguriert ist, den physiologischen Parameter (Pf) des Benutzers basierend auf der Absorption und/oder der Streuung des Infrarotlichts in der Netzhaut (Ru) des Benutzers zu bestimmen.

3. HMM-System (100) nach einem der vorstehenden Ansprüche, das mindestens ein reflektierendes Fenster (15) aufweist, das mit der Kopfmontage (20) verbunden und dazu konfiguriert ist, bei Verwendung vor mindestens einem der Augen des Benutzers eingerichtet zu sein, wenn das HMM-System (10) auf dem Kopf (H) des Benutzers getragen wird, wobei das reflektierende Fenster (15) dazu konfiguriert ist,
es dem Benutzer zu erlauben, eine Außenumgebung (E) durch das reflektierende Fenster (15) zu sehen;
Licht der Lichtquelle (11) auf die Netzhaut (Ru) des Benutzers zu reflektieren und
Licht von der Netzhaut (Ru) zu der Sensorvorrichtung (12) zurück zu reflektieren.

4. HMM-System (10) nach einem der vorstehenden Ansprüche, wobei die Sensorvorrichtung (12) dazu konfiguriert ist, eine jeweilige Lichtintensität (L850, L760) des reflektierten Lichts (Lr) bei mindestens zwei unterschiedlichen Wellenlängen (A) zu messen, wobei die Rechenvorrichtung (13) dazu konfiguriert ist, einen physiologischen Parameter (Pf) zu bestimmen, der einen Richtwert der Oxygenierung basierend auf Vergleichen der jeweiligen Lichtintensität (L850, L760) bei den mindestens zwei unterschiedlichen Wellenlängen (A) angibt.

5. HMM-System (10) nach einem der vorstehenden Ansprüche, wobei die Sensorvorrichtung (12) dazu konfiguriert ist, eine Lichtintensität des reflektierten Lichts (Lr) als Funktion der Zeit (T) zu messen, wobei die Rechenvorrichtung (13) dazu konfiguriert ist, einen physiologischen Parameter (Pf) zu berechnen, der einen Blutdruck des Benutzers basierend auf einer Variation (A) der Lichtintensität angibt.

6. HMM-System (10) nach einem der vorstehenden Ansprüche, wobei die Sensorvorrichtung (12) dazu konfiguriert ist, eine Lichtintensität des reflektierten Lichts (Lr) als Funktion der Zeit (T) zu messen, wobei die Rechenvorrichtung (13) dazu konfiguriert ist, eine Frequenz (F) einer Variation der Lichtintensität zu berechnen, um einen physiologischen Parameter (Pf) zu bestimmen, der eine Herzfrequenz des Benutzers angibt.

7. HMM-System (10) nach einem der vorstehenden Ansprüche, wobei die Rechenvorrichtung (13) ein neuronales Netz oder einen Machine-Learning-Algorithmus aufweist, um einen oder mehrere physiologische Parameter (Pf) basierend auf dem reflektierten Licht (Lr) zu bestimmen.

8. HMM-System (10) nach einem der vorstehenden Ansprüche, das eine Feedback-Vorrichtung aufweist, die dazu konfiguriert ist, ein Feedback-Signal basierend auf dem bestimmten physiologischen Parameter (Pf) auszugeben.

9. Kopfmontiertes Anzeigesystem, HMD-System, (100), mit
dem HMM-System (10) nach einem der vorstehenden Ansprüche;
einem Anzeigegenerator (14), der dazu konfiguriert ist, ein sichtbares Bild (Iv) zu erzeugen, das Informationsinhalt darstellt; und
einem Anzeigeprojektionssystem (15, 16), das dazu konfiguriert ist, das sichtbare Bild (Iv) auf die Netzhaut (Ru) des Benutzers zu projizieren, um dem Benutzer den Informationsinhalt zu vermitteln;
wobei die Sensorvorrichtung (12) dazu konfiguriert ist, Licht (Lr), das von der Netzhaut (Ru) des Benutzers reflektiert wird, unter Verwendung mindestens eines Teils (15) des Anzeigeprojektionssystems (15, 16) zu messen.

10. HMD-System (100) nach dem vorstehenden Anspruch, das einen Strahlteiler/-kombinierer (17) aufweist, der dazu konfiguriert ist,
Licht von dem sichtbaren Bild (Iv), das durch den Anzeigegenerator (5) erzeugt wird, zu der Netzhaut (Ru) des Benutzers durchzulassen; und
Licht (Lr), das von der Netzhaut (Ru) des Benutzers zurück reflektiert wird, zu der Sensorvorrichtung (12) durchzulassen.

11. HMD-System (100) nach dem vorstehenden Anspruch, wobei der Strahlteiler/-kombinierer (17) einen dichroitischen Spiegel aufweist, der dazu konfiguriert ist,
sichtbares Licht des sichtbaren Bildes, das von dem Anzeigegenerator (5) erzeugt wird, zu der Netzhaut (Ru) des Benutzers durchzulassen;
Infrarotlicht, das von der Lichtquelle (11), die eine Infrarotlichtquelle aufweist, erzeugt wird, zur Netzhaut (Ru) des Benutzers durchzulassen;
Infrarotlicht, das von der Netzhaut (Ru) des Benutzers zurück reflektiert wird, zur Sensorvorrichtung (12) durchzulassen

12. HMD-System (100) nach einem der vorstehenden Ansprüche, wobei das Anzeigeprojektionssystem (15, 16) ein reflektierendes Fenster (15) aufweist, das dazu konfiguriert ist,
es dem Benutzer zu erlauben, durch das reflektierende Fenster (15) zu sehen;
das sichtbare Bild (Iv) von dem Anzeigegenerator (14) zur Projektion des sichtbaren Bildes auf die Netzhaut (Ru) des Benutzers zu reflektieren; und
Licht von der Netzhaut (Ru) des Benutzers zu dem Überwachungssystem (10) zur Messung durch die Sensorvorrichtung (12) zu reflektieren.

13. Steuersystem zum Steuern eines Fahrzeugs, wobei das Steuersystem Folgendes umfasst
das HMM-System (10) oder HMD-System (100) nach einem der vorstehenden Ansprüche, das dazu konfiguriert ist, einen physiologischen Parameter (Pf) eines Benutzers, der das Fahrzeug bedient, zu bestimmen; und
einen Controller, der dazu konfiguriert ist, eine Angabe des bestimmten physiologischen Parameters (Pf) zu empfangen und die Steuerung des Fahrzeugs basierend auf der empfangenen Angabe anzupassen.

14. Verfahren zum Überwachen eines physiologischen Parameters (Pf) unter Verwendung eines kopfmontierten Überwachungssystems, HMM-Systems, (10) nach einem der Ansprüche 1 bis 8, während das HMM-System auf dem Kopf (H) eines Benutzers getragen wird, wobei ein teilweise reflektierendes Fenster (15) des HMM-Systems vor den Augen des Benutzers eingerichtet ist, während es dem Benutzer erlaubt wird, seine Umgebung frei durch das teilweise reflektierende Fenster (15) zu sehen und/oder ein sichtbares Bild (Iv) zu sehen, das Informationsinhalt darstellt, das über das teilweise reflektierende Fenster (15) auf die Netzhaut (Ru) des Benutzers innerhalb des Auges des Benutzers projiziert wird, wobei das Verfahren Folgendes umfasst
Projizieren durch ein Projektionssystem (16, 18) von Quelllicht (Ls) von einer Lichtquelle (11) zu der Netzhaut (Ru) des Benutzers über das teilweise reflektierende Fenster (15) des HMM-Systems, das vor dem Auge des Benutzers eingerichtet ist, und über eine Pupille (Pu) des Benutzers des Auges des Benutzers, wobei die Pupille (Pu) des Benutzers an einer ersten Pupillenebene (Pp) des Projektionssystems (16, 18) eingerichtet ist;
Bestimmen durch Pupillenverfolgungsmittel einer Bildposition (X') der Pupille (Pu) des Benutzers in einer zweiten Pupillenebene (Pp') des Projektionssystems (16, 18) konjugiert mit der ersten Pupillenebene (Pp);
Messen durch eine Sensorvorrichtung (12) von Licht (Lr), das von der Netzhaut (Ru) des Benutzers reflektiert wird;
Bestimmen eines physiologischen Parameters (Pf) des Benutzers basierend auf dem gemessenen Licht (Lr);
wobei das gemessene Licht (Lr), das zum Bestimmen des physiologischen Parameters (Pf) verwendet wird, ausschließlich von der Position (X) der Pupille (Pu) des Benutzers stammt, während Licht, das von umgebenden Teilen des Auges des Benutzers reflektiert wird, weggelassen oder herausgefiltert wird durch mindestens eines von:
räumlichen Filtern von Licht, das durch die zweite Pupillenebene (Pp') von der Lichtquelle (11) zu der Netzhaut (Ru) des Benutzers durchgeht, und/oder von der Netzhaut (Ru) des Benutzers zu der Sensorvorrichtung (12), um ausschließlich Licht durchzulassen, das mit der bestimmten Bildposition (X') der Pupille (Pu) des Benutzers zusammenfällt,
ausschließlichem Erzeugen des Quelllichts (Ls) an der bestimmten Bildposition (X') der Pupille (Pu) des Benutzers unter Verwendung der Lichtquelle (11), die an der zweiten Pupillenebene (Pp') bereitgestellt ist,
ausschließlichem Messen des reflektierten Lichts (Lr) an der bestimmten Bildposition (X') der Pupille (Pu) des Benutzers unter Verwendung der Sensorvorrichtung (12), die an der zweiten Pupillenebene (Pp') bereitgestellt ist.

## Revendications

1. Système (10) de surveillance monté sur la tête, HMM, comprenant
un support de tête (20) configuré pour fixer le système HMM (10) à la tête (H) d'un utilisateur ;
une source lumineuse (11) configurée pour projeter une lumière source (Ls) sur la rétine (Ru) d'un utilisateur lorsque le système HMM est fixé à la tête (H) de l'utilisateur par le support de tête (20) ;
un dispositif capteur (12) configuré pour mesurer la lumière réfléchie (Lr) réfléchie par la rétine (Ru) de l'utilisateur ;
un système de projection (16, 18) configuré pour projeter la lumière source (Ls) de la source lumineuse (11) sur un œil de l'utilisateur, au moins une partie de la lumière source (Ls) atteignant la rétine (Ru) de l'utilisateur, à l'intérieur de l'œil, par l'intermédiaire d'une pupille (Pu) de l'utilisateur, de l'œil, dans lequel la pupille (Pu) de l'utilisateur est disposée à un premier plan pupillaire (Pp) du système de projection (16, 18) ;
des moyens de suivi de pupille configurés pour
déterminer une position d'image (X') de la pupille (Pu) de l'utilisateur dans un deuxième plan pupillaire (Pp') du système de projection (16, 18), conjugué au premier plan pupillaire (Pp), et
utiliser la lumière réfléchie (Lr) provenant exclusivement de la position (X) de la pupille (Pu) de l'utilisateur pour mesurer l'absorption et/ou la diffusion de la lumière source (Ls) par la rétine (Ru) de l'utilisateur, tout en omettant ou en filtrant la lumière réfléchie par les parties environnantes de l'œil de l'utilisateur, par au moins l'un parmi
le filtrage spatial de la lumière passant par le deuxième plan pupillaire (Pp') depuis la source lumineuse (11) vers la rétine (Ru) de l'utilisateur, et/ou depuis la rétine (Ru) de l'utilisateur vers le dispositif capteur (12), afin de ne laisser passer que la lumière coïncidant avec la position d'image (X') déterminée de la pupille (Pu) de l'utilisateur,
la fourniture d'une source lumineuse (11) au deuxième plan pupillaire (Pp') pour générer exclusivement la lumière source (Ls) à la position d'image (X') déterminée de la pupille (Pu) de l'utilisateur,
la fourniture d'un dispositif capteur (12) au deuxième plan pupillaire (Pp') pour mesurer exclusivement la lumière réfléchie (Lr) à la position d'image (X') déterminée de la pupille (Pu) de l'utilisateur ; et
un dispositif informatique (13) configuré pour surveiller en continu un paramètre physiologique (Pf) de l'utilisateur sur la base de la lumière mesurée (Lr).

2. Système HMM (10) selon la revendication 1,
dans lequel la source lumineuse (11) comprend une source lumineuse infrarouge configurée pour générer une lumière infrarouge éclairant la rétine (Ru) de l'utilisateur ;
dans lequel le dispositif capteur (12) est configuré pour mesurer une partie de la lumière infrarouge générée (Lr) réfléchie en retour par la rétine (Ru) de l'utilisateur en fonction au moins en partie de l'absorption et/ou de la diffusion de la lumière infrarouge dans la rétine (Ru) de l'utilisateur ;
dans lequel le dispositif informatique (13) est configuré pour déterminer le paramètre physiologique (Pf) de l'utilisateur sur la base de l'absorption et/ou de la diffusion de la lumière infrarouge dans la rétine (Ru) de l'utilisateur.

3. Système HMM (100) selon l'une quelconque des revendications précédentes, comprenant au moins une fenêtre réfléchissante (15), reliée au support de tête (20), et configurée pour être, en utilisation, disposée devant au moins l'un des yeux de l'utilisateur lorsque le système HMM (10) est porté sur la tête (H) de l'utilisateur, dans lequel la fenêtre réfléchissante (15) est configurée pour
permettre à l'utilisateur de voir un environnement externe (E) à travers la fenêtre réfléchissante (15) ;
réfléchir la lumière de la source lumineuse (11) sur la rétine (Ru) de l'utilisateur ; et
réfléchir la lumière provenant de la rétine (Ru) de l'utilisateur vers le dispositif capteur (12).

4. Système HMM (10) selon l'une quelconque des revendications précédentes, dans lequel le dispositif capteur (12) est configuré pour mesurer une intensité lumineuse respective (L850, L760) de la lumière réfléchie (Lr) à au moins deux longueurs d'onde distinctes (λ), dans lequel le dispositif informatique (13) est configuré pour déterminer un paramètre physiologique (Pf) comprenant une valeur indicative de l'oxygénation sur la base de la comparaison de l'intensité lumineuse respective (L850, L760) aux au moins deux longueurs d'onde distinctes (λ).

5. Système HMM (10) selon l'une quelconque des revendications précédentes, dans lequel le dispositif capteur (12) est configuré pour mesurer une intensité lumineuse de la lumière réfléchie (Lr) en fonction du temps (T), dans lequel le dispositif informatique (13) est configuré pour calculer un paramètre physiologique (Pf) indiquant une pression sanguine de l'utilisateur sur la base d'une variation (A) de l'intensité lumineuse.

6. Système HMM (10) selon l'une quelconque des revendications précédentes, dans lequel le dispositif capteur (12) est configuré pour mesurer une intensité lumineuse de la lumière réfléchie (Lr) en fonction du temps (T), dans lequel le dispositif informatique (13) est configuré pour calculer une fréquence (F) d'une variation de l'intensité lumineuse afin de déterminer un paramètre physiologique (Pf) indiquant une fréquence cardiaque de l'utilisateur.

7. Système HMM (10) selon l'une quelconque des revendications précédentes, dans lequel le dispositif informatique (13) comprend un réseau de neurones ou un algorithme d'apprentissage automatique pour déterminer un ou plusieurs paramètres physiologiques (Pf) sur la base de la lumière réfléchie (Lr).

8. Système HMM (10) selon l'une quelconque des revendications précédentes, comprenant un dispositif de rétroaction configuré pour fournir en sortie un signal de rétroaction sur la base du paramètre physiologique (Pf) déterminé.

9. Système (100) d'affichage monté sur la tête, HMD, comprenant
le système HMM (10) selon l'une quelconque des revendications précédentes ;
un générateur d'affichage (14) configuré pour générer une image visible (Iv) représentant un contenu informationnel ; et
un système de projection d'affichage (15, 16) configuré pour projeter l'image visible (Iv) sur la rétine (Ru) de l'utilisateur afin de transmettre le contenu informationnel à l'utilisateur ;
dans lequel le dispositif capteur (12) est configuré pour mesurer la lumière (Lr) réfléchie par la rétine (Ru) de l'utilisateur à l'aide d'au moins une partie (15) du système de projection d'affichage (15, 16).

10. Système HMD (100) selon la revendication précédente, comprenant un séparateur/combineur de faisceaux (17) configuré pour
laisser passer la lumière de l'image visible (Iv) générée par le générateur d'affichage (5) vers la rétine (Ru) de l'utilisateur ; et
laisser passer la lumière (Lr) réfléchie en retour par la rétine (Ru) de l'utilisateur vers le dispositif capteur (12).

11. Système HMD (100) selon la revendication précédente, dans lequel le séparateur/combineur de faisceaux (17) comprend un miroir dichroïque configuré pour
laisser passer la lumière visible de l'image visible générée par le générateur d'affichage (5) vers la rétine (Ru) de l'utilisateur ;
laisser passer la lumière infrarouge générée par la source lumineuse (11), comprenant une source lumineuse infrarouge, vers la rétine (Ru) de l'utilisateur ;
laisser passer la lumière infrarouge réfléchie en retour par la rétine (Ru) de l'utilisateur vers le dispositif capteur (12).

12. Système HMD (100) selon l'une quelconque des revendications précédentes, dans lequel le système de projection d'affichage (15, 16) comprend une fenêtre réfléchissante (15) configurée pour
permettre à l'utilisateur de voir à travers la fenêtre réfléchissante (15) ;
réfléchir l'image visible (Iv) provenant du générateur d'affichage (14) en vue de la projection de l'image visible sur la rétine (Ru) de l'utilisateur ; et
réfléchir la lumière provenant de la rétine (Ru) de l'utilisateur vers le système de surveillance (10) en vue d'une mesure par le dispositif capteur (12).

13. Système de commande pour commander un véhicule, le système de commande comprenant
le système HMM (10) ou le système HMD (100) selon l'une quelconque des revendications précédentes, configuré pour déterminer un paramètre physiologique (Pf) d'un utilisateur actionnant le véhicule ; et
un contrôleur configuré pour recevoir une indication du paramètre physiologique (Pf) déterminé et adapter la commande du véhicule sur la base de l'indication reçue.

14. Procédé de surveillance d'un paramètre physiologique (Pf) au moyen d'un système (10) de surveillance monté sur la tête, HMM, selon l'une quelconque des revendications 1 à 8, pendant que le système HMM est porté sur la tête (H) d'un utilisateur, avec une fenêtre partiellement réfléchissante (15) du système HMM disposée devant les yeux de l'utilisateur, tout en permettant à l'utilisateur de voir librement son environnement à travers ladite fenêtre partiellement réfléchissante (15) et/ou de voir une image visible (Iv) représentant un contenu informationnel projeté par l'intermédiaire de ladite fenêtre partiellement réfléchissante (15) sur la rétine (Ru) de l'utilisateur à l'intérieur de l'œil de l'utilisateur, le procédé comprenant le fait de
projeter, par le biais d'un système de projection (16, 18), une lumière source (Ls), provenant d'une source lumineuse (11), sur la rétine (Ru) de l'utilisateur, par l'intermédiaire de ladite fenêtre partiellement réfléchissante (15) du système HMM disposée devant l'œil de l'utilisateur, et par l'intermédiaire d'une pupille (Pu) de l'utilisateur de l'œil de l'utilisateur, dans lequel la pupille (Pu) de l'utilisateur est disposée à un premier plan pupillaire (Pp) du système de projection (16, 18) ;
déterminer, par le biais de moyens de suivi de pupille, une position d'image (X') de la pupille (Pu) de l'utilisateur dans un deuxième plan pupillaire (Pp') du système de projection (16, 18), qui est conjugué au premier plan pupillaire (Pp) ;
mesurer, par le biais d'un dispositif capteur (12), la lumière (Lr) réfléchie par la rétine (Ru) de l'utilisateur ;
déterminer un paramètre physiologique (Pf) de l'utilisateur sur la base de la lumière mesurée (Lr) ;
dans lequel la lumière mesurée (Lr) utilisée pour la détermination du paramètre physiologique (Pf) provient exclusivement de la position (X) de la pupille (Pu) de l'utilisateur, tandis que la lumière réfléchie par les parties environnantes de l'œil de l'utilisateur est omise ou filtrée, par au moins l'un parmi :
le filtrage spatial de la lumière passant par le deuxième plan pupillaire (Pp') depuis la source lumineuse (11) vers la rétine (Ru) de l'utilisateur, et/ou depuis la rétine (Ru) de l'utilisateur vers le dispositif capteur (12), afin de ne laisser passer que la lumière coïncidant avec la position d'image (X') déterminée de la pupille (Pu) de l'utilisateur,
la génération exclusive de la lumière source (Ls) à la position d'image (X') déterminée de la pupille (Pu) de l'utilisateur à l'aide de la source lumineuse (11) fournie au deuxième plan pupillaire (Pp'), et
la mesure exclusive de la lumière réfléchie (Lr) à la position d'image (X') déterminée de la pupille (Pu) de l'utilisateur à l'aide du dispositif capteur (12) fourni au deuxième plan pupillaire (Pp').
